# EUROPEAN PATENT APPLICATION

(11) **EP 2 008 531 A1**
(43) Date of publication of application: **31.12.2008**
(21) Application number: 07738558.1
(22) Date of filing: 14.03.2007
(51) Int. Cl.: A23L 1/30

(54) **COMPOSITION FOR FOODS AND DRINKS HAVING IMPROVED HYGROSCOPICITY**

(30) Priority: 15.03.2006 JP 2006071026
(71) Applicant: Suntory Limited, Osaka-shi Osaka 530-8203 (JP)
(72) Inventor: The designation of the inventor has not yet been filed
(74) Representative: Vossius & Partner
(86) International application number: PCT/JP2007/055088
(87) International publication number: WO 2007/105757

(57) **Abstract**

The object of the present invention is to provide a composition for food and beverage comprising a hygroscopic powdery material having improved hygroscopicity as well as food and beverage containing the composition.

The present invention provides compositions for food and beverage comprising a hygroscopic powdery material and a sesamin-class compound(s), more specifically powdery compositions for food and beverage comprising a hygroscopic powdery material and a sesamin-class compound(s) mixed with each other wherein the hygroscopic powdery material has improved hygroscopicity as well as foods and beverages containing the compositions.

## Description

### TECHNICAL FIELD

The present invention relates to compositions for food and beverage comprising a hygroscopic powdery material and a sesamin-class compound(s), more specifically powdery compositions for food and beverage comprising a hygroscopic powdery material and a sesamin-class compound(s) mixed with each other, thereby improving hygroscopicity of the hygroscopic powdery material, as well as foods and beverages containing the compositions.

### BACKGROUND ART

The absorption of moisture by powdery materials often becomes a concern during manufacturing and/or during storage. The degree of hygroscopicity varies with the material, but in the most serious cases, powder absorbs water to form a solution (deliquesces). If moisture absorption occurs during manufacturing of a powdery material, it may cause problems such as changes in flow characteristics, increase in viscosity, aggregation, and decrease in releasability, thereby inviting not only difficulty in handling but also loss in chemical stability resulting in fluctuation in the composition of the material or product. If moisture absorption occurs during storage of a powder material, it may cause problems not only such as deterioration of appearance but also decrease in chemical stability of active ingredients contained, or changes in solubility due to changes in the crystal form. Various methods have been proposed to solve the problems of hygroscopicity or deliquescence of hygroscopic powdery materials that are difficult to handle during manufacturing or storage as described above, e.g., salts of organic or inorganic acids, inorganic salts and compound materials used in drugs or the like.

For example, a method for preventing deliquescence or agglomeration by adding powdery fat and oil and calcium carbonate to a powdery material (patent document 1), a method for preventing agglomeration by adding a sucrose fatty acid ester (patent document 2), a method for preventing deliquescence by adsorbing a deliquescent material into an adsorbent material (patent document 3), an improved product wherein a hygroscopic powdery material is coated with an enteric coat (patent document 4), and a method for retarding deliquescence by changing the crystal form (patent document 5), and other approaches have been disclosed.

Naturally occurring L-carnitine is a special amino acid known as an essential nutrient for burning fat in the body to convert it into energy and expected to help: preventing life-style related diseases by burning excess fat; recovering from fatigue or increasing stamina; improving performance in sports or in daily life;, or increasing the basal metabolism. Recently, L-carnitine has been used in foods and beverages such as dietary supplements, products for diet therapy, and health foods or compositions for food and beverage in order to appropriately take it because the amount of L-carnitine present in the body decreases and becomes insufficient with age after it reaches a peak in the twenties. However, L-carnitine and derivatives thereof are known to have very high hygroscopicity and to be very unstable when they form an inner salt (or a betaine compound) represented by the formula below:

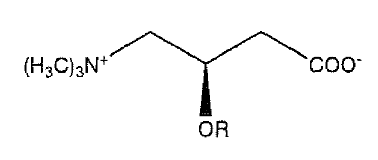

wherein R is H or a C₁-C₅ lower alkanoyl.

When L-carnitine absorbs moisture, it turns into a paste or a glue and loses chemical stability to release trimethylamine and produces an unpleasant fishy odor. For these reasons, methods for decreasing hygroscopicity of L-carnitine or a salt thereof or a derivative thereof have been proposed. For example, a process for preparing carnitine orotate with low hygroscopicity (patent document 6), the use of maleate and fumarate of L(-)carnitine (patent document 7), sulfate and oxalate of L(-)carnitine (patent document 8), and the use of L(-)carnitine L(+)tartrate (2:1) (patent document 9), and other approaches have been disclosed.
Patent document
1. JP 2001-224319-A
2. JP 2004-121175-A
3. JP 2003-95980-A
4. JP 2004-35505-A
5. JP 2000-342902-A
6. Japanese Patent No. 303067
7. US Patent No. 4602039
8. French Patent No. 2 529 545 (Application No. FR 82 11626)
9. European Patent No. 0434088

### DISCLOSURE OF THE INVENTION

### PROBLEMS TO BE SOLVED BY THE INVENTION

As described above, methods for improving hygroscopicity and deliquescence of various hygroscopic powdery materials including L-carnitine have been proposed. However, no satisfactory method has existed that can be easily applied and prevents or inhibits hygroscopicity. Approaches for improving hygroscopicity by additives gave rise to concerns about safety and side effects of the additives, as well as problems of flavor when they were used in food and beverage. Moreover, methods used for improving hygroscopicity by modifying the crystal form or salt form of a powdery material gave rise to manufacturing problems such as making the processes for manufacturing, more complex. For example, the composition described in patent document 9 above was prepared by adding an L-carnitine inner salt to L(+)tartaric acid boiled in 90% aqueous ethanol, thus requiring a lot of a carnitine salt-containing solution to be concentrated at a high temperature under reduced pressure, which involved not only considerable energy wasting but also serious problems such as cost increase, recycling of solvents, environmental pollution and disposal of toxic wastes when organic solvents were used.

Accordingly, an object of the present invention is to provide a composition for food and beverage comprising a hygroscopic powdery material, wherein hygroscopicity of the hygroscopic powdery material, especially carnitine or a salt thereof or a derivative thereof has been improved by a method that can be easily and industrially applied at a low cost. MEANS FOR SOLVING PROBLEM

As a result of careful studies to solve the problems described above, the inventors found that hygroscopicity of hygroscopic powdery materials is improved by mixing the hygroscopic powdery materials with powdery sesamin-class compounds, and especially when the hygroscopic powdery material is carnitine or a salt thereof or a derivative thereof, its hygroscopicity is remarkably improved. Then, we verified that a powdery sesamin-class compound(s) are readily applicable to food and beverage because they are tasteless and odorless, and thus accomplished the present invention.

Accordingly, the present invention provides a composition for food and beverage, preferably a powdery composition for food and beverage, comprising a hygroscopic powdery material and a sesamin-class compound(s), preferably a powdery sesamin-class compound(s).

The present invention also provides a powdery composition for food and beverage comprising a hygroscopic powdery material and a sesamin-class compound(s) mixed with each other wherein the hygroscopic powdery material has improved hygroscopicity.

The present invention also provides the composition wherein the hygroscopic powdery material is carnitine or a salt thereof or a derivative thereof. The present invention also provides the composition wherein the sesamin-class compound(s) are sesamin and/or episesamin, more specifically sesamin and episesamin.

The present invention also provides a food or beverage containing any one of the compositions.

### BRIEF DESCRIPTION OF THE DRAWING

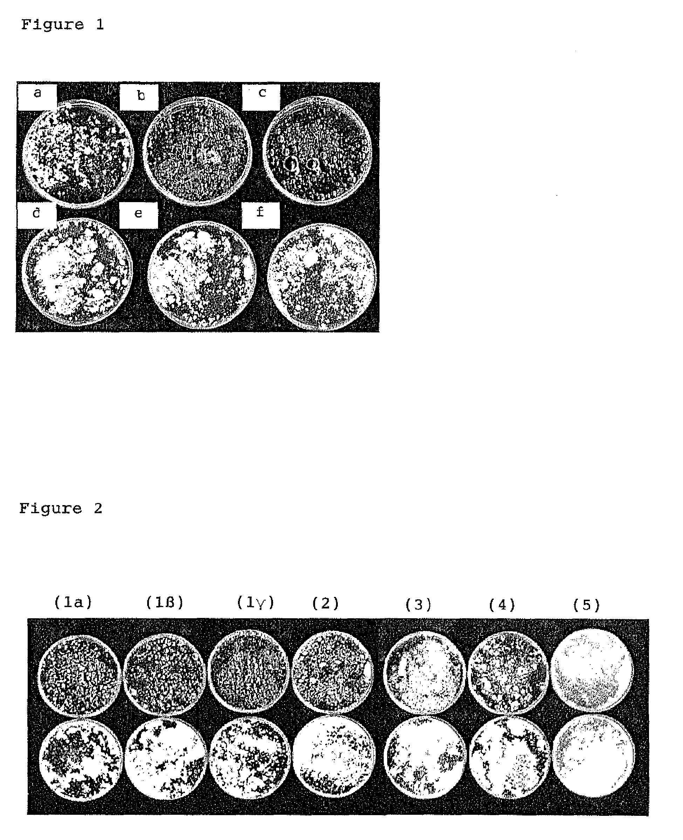

Figure 1 shows the results of improvement in the hygroscopicity of carnitine when carnitine was mixed with sesamin-class compounds in Example 1. (a) Carnitine alone , (b) Carnitine alone, (c) Carnitine alone, (d) Carnitine + Sesamin, (e) Carnitine + Sesamin, (f) Carnitine + Sesamin.
Figure 2 shows the results of improvement in the hygroscopicity of carnitine when carnitine was mixed with various additives in Example 1. Upper line: After 12 hours at 40°C/75%RH, Lower line: After 12 hours at 20°C. Comparative example 1: (1a) a-Cyclodextrin, (1β) β-Cyclodextrin, and (1γ) γ-Cyclodextrin, Comparative example 2: (2) SiO₂, Comparative example 3: (3) Starch, Comparative example 4: (4) Water-soluble dietary fiber, Comparative example 5: (5) Sucrose fatty acid ester.

### BEST MODE FOR CARRYING OUT THE INVENTION

As used herein, "hygroscopicity" means the property possessed by a material of absorbing water vapor in the atmosphere when it is exposed to the air, and especially, the property of absorbing water vapor in the atmosphere which allows a material to dissolve in absorbed water, called deliquescence is also included in the term "hygroscopicity" used herein. The expression "improved hygroscopicity" of a hygroscopic powdery material means that hygroscopicity decreases, or deliquescence and/or agglomeration is prevented, or deliquescence and/or agglomeration is inhibited, as compared with untreated hygroscopic powdery materials.

Hygroscopicity can be measured and/or evaluated by a method known to those skilled in the art e.g., as described in the Example below. Specifically, a suitable method involves allowing a weighed material to stand under given conditions such as room temperature or high temperature and high humidity for a suitable time, and then visually observing the changes (for e.g., observing moisture on the surface of the material, observing disappearance of solids, observing fluidity or the degree of agglomeration of powder, observing color changes, etc.) or measuring weight gain (the amount of absorbed water).

As used herein, the "hygroscopic powdery material" may be any material acceptable for use in foods and beverages or drugs in which the hygroscopicity becomes a concern during manufacturing and/or storage or other stage of a desired product. As used herein, the material in which "hygroscopicity becomes a concern" can be readily determined by those skilled in the art by reference to for e.g., the description about hygroscopicity herein, specifically moisture absorption during manufacturing or storage of a product. Especially, it is a material showing hygroscopicity (or deliquescence) under conditions of temperature 40°C, relative humidity 75% used in acceleration test for determining storage stability of a product. Specific examples of hygroscopic powdery materials include: salts of organic or inorganic acids such as sodium metaphosphate, potassium pyrophosphate, sodium acetate, ammonium alum; inorganic salts such as magnesium chloride, calcium chloride; sugars such as xylooligosaccharide; and active ingredients used in medicines, e.g., sodium valproate, suplatast tosilate, choline salts, rivastigmine, pyridostigmine bromide, bacampicillin, L-carnitine and derivatives thereof, moricizine hydrochloride, and herbal extracts such as Quercus salicina extract.

The hygroscopic powdery material of the present invention is preferably a hygroscopic material with minimal problem of flavor so far as possessing similar physiological activity because it is contained in a composition for a food or a beverage. For example, when the hygroscopic material is carnitine or a salt thereof or a derivative thereof, it is more preferable to use tasteless and odorless L-carnitine than L-carnitine tartrate having a strong acidic taste.

As used herein, carnitine or a salt thereof or a derivative thereof includes carnitine [(CH₃)₃N⁺CH₂CH(OH)CH₂COO⁻, γ-trimethylammonium-β-hydroxybutyric acid], isomers thereof and salts thereof, as well as derivatives of carnitine, isomers thereof and salts thereof (e.g., derivatives converted in vivo into carnitine). Examples include carnitine, L-carnitine, carnitine chloride (DL-, L-isomers), L-carnitine tartrate, L-carnitine maleate, L-carnitine fumarate, L-carnitine sulfate, L-carnitine oxalate, magnesium salt of L-carnitine, acyl-L-carnitine, acetyl-L-carnitine, propionyl-L-carnitine, carnitine orotate, etc. These are not limited by the preparation process, and may be naturally extracted, fermented from microorganisms or synthesized, but a preferred example is a naturally occurring L-carnitine or a salt thereof or a derivative thereof, for e.g., represented by the formula below:

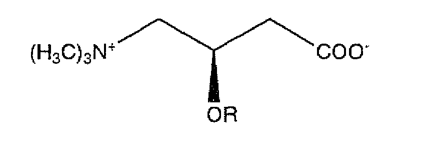

wherein R is H or a C₁-C₅ lower alkanoyl. The explanation about carnitine herein also applies to salts of carnitine and derivatives of carnitine or salts thereof.

As used herein, the "sesamin-class compound(s)" includes sesamin and its analogs, specifically sesamin, sesaminol, episesaminol, sesamolin, episesamin, dioxabicyclo [3.3.0] octane derivatives (e.g., described in JP Hei 04-9331-A), etc. Especially, sesamin and episesamin are preferred in the present invention. Moreover, the "sesamin-class compound(s)" also includes glycosides of sesamin and its analogs as well as their metabolites, and any of them may be contained in the compositions for food and beverage of the present invention so far as they are suitable for providing the compositions for food and beverage of the present invention.

The sesamin-class compound(s) used in the present invention are not limited by the preparation process or the like, so far as they are solid. When sesamin and/or episesamin is used as a sesamin-class compound(s), for example, sesamin extracted from sesame oil by a publicly known method (e.g., extracting sesame oil with hot methanol, and after removal of methanol, extracting the residue with acetone (described in JP Hei 04-9331-A)) (also referred to as sesamin extracts or purified sesamin) can be used. The sesame oil used as raw material is not specifically limited, but sesamin extracts or purified sesamin extracted from tasteless and odorless sesame oil are preferably used because the flavor specific to sesame oil is sometimes evaluated as organoleptically unfavorable when they are contained in the compositions for food and beverage of the present invention. Besides, if the preferred sesamin content described below is to be included by using a low-purity sesamin-class compound(s), the volume of the resulting compositions for food and beverage or the resulting foods and beverages may excessively increase to cause difficulty in consuming. Especially, formulations for oral administration (tablets, capsules, etc.) become bulky so as to cause difficulty in consuming. Thus, purified sesamin is preferably used because it may be taken in low doses.

The sesamin-class compound(s) of the present invention are preferably a powdery sesamin-class compound(s) in view of the improvement of hygroscopicity of the hygroscopic powdery material. The particle size of the powdery sesamin-class compound(s) may be normally in the range of JIS Standard Sieve 10-250 mesh, preferably 60-200 mesh, depending on the method by which they are mixed with the hygroscopic powdery material (homogeneity).

When the hygroscopic powdery material included in combination with the sesamin-class compound in the compositions for food and beverage of the present invention has health-enhancing or other effects, the sesamin-class compound is preferably inter alia sesamin and/or episesamin. This is because various physiological effects of these sesamin-class compounds may be produced additively or synergistically with the effects of the hygroscopic material. When the hygroscopic powdery material is for e.g., carnitine, the energy metabolism-promoting effect and motor ability-promoting effect of carnitine may be produced additively or synergistically with one of the physiological effects of sesamin and episesamin such as energy metabolism-promoting effect (Biosci. Biotechnol. Biochem., 69, 179-188, 2005) or motor ability-promoting effect (Int. J. Sports Med., 24, 530-534, 2003, Biofactors, 21, 191-196, 2004), thereby providing excellent health foods.

The compositions for food and beverage of the present invention comprise a hygroscopic powdery material and a sesamin-class compound(s) and can be obtained by mixing the hygroscopic powdery material and the sesamin-class compound(s), optionally with other additives. The manner of mixing is not limited, and any method can be used so far as the materials are homogeneously mixed. Suitable methods include mixing with commonly known mixers, e.g., horizontal cylindrical mixers, V-shaped mixers, double cone mixers, cube mixers, screw mixers, ribbon mixers, media agitation mills, high-speed rotary grinding/impact mills, and pneumatic grinders such as jet mills; grinding/mixing in mortars or ball mills; and agitation/mixing in bags made from polyethylene (PE), nylon or the like bags. In view of easy operation without requiring a special machine, agitation/mixing in a bag can be preferably used. When the hygroscopic powdery material and a sesamin-class compound(s) are thoroughly mixed, hygroscopicity can be further improved.

In the compositions for food and beverage of the present invention, the amount of the sesamin-class compound(s) depends on the type of the hygroscopic material used in combination and the presence or absence of other additives etc., but it is suitably contained in an amount equivalent to a weight ratio to the hygroscopic material of about 0.001-100:100, preferably 0.01-10:100.

The compositions for food and beverage of the present invention can be directly used as foods and beverages described below, or contained as appropriate in the foods and beverages by a method known to those skilled in the art. The compositions for food and beverage of the present invention are chemically stable and suitable for storage or manufacturing foods and beverages because of the improved hygroscopicity of the powdery material contained in the composition.

As used herein, food and beverage means to include seasonings, dietary supplements, health foods, foods for diet therapy, total health foods, supplements and drinks as well as medicines for oral administration. The foods and beverages of the present invention can be solid (e.g., crystals, capsules, tablets, powder), semi-solid (e.g., gel, paste), and liquid (e.g., mineral water, soft drinks, fruit drink, isotonic drink, liquors). Preferably, the foods and beverages of the present invention are in the form of tablets or granules, or tablets or granules to be dissolved in liquid just before ingestion.

The foods and beverages of the present invention can be prepared by including a composition for food and beverage of the present invention using a method known to those skilled in the art. The foods and beverages of the present invention can be expected to stably produce desired effects of the hygroscopic powdery material and sesamin.

The amounts of the hygroscopic powdery material and sesamin in the foods and beverages of the present invention are not specifically limited, but can be determined as appropriate by those skilled in the art depending on the manner of taking the foods and beverages on the basis of their preferred daily doses.

In addition to the hygroscopic powdery material and sesamin-class compound(s) mentioned above, the compositions for food and beverage of the present invention may contain optional additives. In addition to the compositions for food and beverage of the present invention mentioned above, the foods and beverages of the present invention may contain any ingredient used in conventional foods and beverages. Examples of these additives and/or ingredients include vitamins such as vitamin E, vitamin C; sugars, excipients, disintegrants, binders, lubricants, emulsifiers, tonicity agents (isotonizing agents), buffers, solubilizers, preservatives, stabilizers, antioxidants, colorants, flavoring agents, perfumes, coagulants, pH-modifiers, thickeners, extract powders, herbal medicines, inorganic salts, etc.

Further, the compositions for food and beverage or the foods and beverages of the present invention can be provided with indications of their specific purposes (e.g., to supplement nutrition, maintain health, burn fat, protect against obesity, keep in shape after slimming, etc.) and/or specific directions (e.g., dose, frequency, manner, etc.).

### EXAMPLES

The following examples further illustrate the present invention without, however, limiting the invention thereto.

### Example 1: Improvement in the hygroscopicity of carnitine

Carnitine was used as a hygroscopic material to evaluate the improvement in the hygroscopicity of carnitine by mixing it with sesamin-class compounds.

### Experimental materials

L-carnitine crystalline powder (L-carnitine crystalline powder from San-Ei Gen F.F.I., Inc.) was used as a hygroscopic powdery material. A mixture of purified powdery sesamin and episesamin (sesamin:episesamin=51.1:48.2 (weight ratio)) was used as sesamin-class compounds.
As comparative examples, the following additives frequently used in formulations were used in place of the sesamin-class compound(s) to evaluate the improvement in the hygroscopicity of carnitine.
Comparative example 1: Cyclodextrins (a-CD / β-CD / γ-CD) (DexyPearl a-100 / β-100 / γ-100 from Ensuiko Sugar Refining Co., Ltd.)
Comparative example 2: SiO₂ (Sylopage from Fuji Silysia Chemical Ltd.)
Comparative example 3: Starch (Perfiller 102 from Nippon Starch Chemical Co., Ltd.)
Comparative example 4: Water-soluble dietary fiber (Sunfiber from Taiyo Kagaku Co., Ltd.)
Comparative example 5: Sucrose fatty acid ester (DK ester F-10 from Dai-ichi Kogyo Seiyaku Co., Ltd.).

### Experimental procedure

1. In a petri dish (40f), 200 mg of powder of carnitine or a 1:1 mixture of carnitine and the sesamin-class compounds (mixed in a PE bag) was placed.
2. Changes in aspects were visually observed after the petri dish was covered and allowed to stand (A) at room temperature (20°C) or (B) in an incubator (40°C, 75%RH) for 12 hours.
3. Powders of Comparative examples 1-5 were used in place of the sesamin-class compounds and evaluated in the same manner.

### Results

Figure 1 shows the results of improvement in the hygroscopicity of carnitine when carnitine was mixed with the sesamin-class compounds. Carnitine alone deliquesced (absorbed moisture) not only at high temperature and high humidity (40°C, 75%RH) but also at room temperature because of high hygroscopicity (Figure 1 (b) (c)). On the other hand, the mixture of equal amounts of the sesamin-class compounds and carnitine remained powdery at both room temperature and high temperature and high humidity (Figure 1 (e) (f)). Thus, it was shown that the sesamin-class compounds have the effect of improving hygroscopicity (deliquescence) of the hygroscopic material. When mixing conditions of carnitine and sesamin changed, it was shown that the effect of improving hygroscopicity increases with the extent to which they are mixed (experimental results not shown).

Figure 2 shows the results of improvement in the hygroscopicity of carnitine when carnitine was mixed with additives of Comparative examples 1-5. Cyclodextrins (Comparative example 1, Figure 2 (1a), (1β), (1γ)) and SiO₂ (Comparative example 2, Figure 2 (2)) showed no effect of improving hygroscopicity. Starch (Comparative example 3, Figure 2 (3)) and water-soluble dietary fiber (Comparative example 4, Figure 2 (4)) partially showed deliquescence. Sucrose fatty acid ester (Comparative example 5, Figure 2 (5)) showed improvement in hygroscopicity.

These results suggested that the sesamin-class compounds or sucrose fatty acid ester is effective in improving hygroscopicity of carnitine. It seemed to be especially preferable to use sesamin-class compounds because they are tasteless and odorless.

### Example 2: Improvement in the hygroscopicity of various hygroscopic powdery materials

L-carnitine L-tartrate (L-carnitine L-tartrate S from San-Ei Gen F.F.I., Inc., which is improved in hygroscopicity as compared with L-carnitine, but still hygroscopic), MgCl₂ (Magnesium chloride S from Tomita Pharmaceutical Co., Ltd.), and xylooligosaccharide (Xylooligo-95P from Suntory Limited) were used as hygroscopic powdery materials. The effect of sesamin-class compounds on improving the hygroscopicity of these hygroscopic powdery materials was evaluated by the same procedure as described in Example 1. The results showed that all of the hygroscopic powdery materials improved in hygroscopicity.

### Example 3: Formulation examples

In the following preparation examples, the sesamin-class compounds and carnitine described in Example 1 are used.

### (Formulation example 1) Granules

| | |
|---|---|
| Sesamin-class compounds | 0.01 g |
| Carnitine | 100 g |
| Tocopherol acetate | 0.25 g |
| Silicic anhydride | 20.5 g |
| Corn starch | 79.0 g |

The powdery materials are homogeneously mixed and then combined with 100 ml of a 10% hydroxypropylcellulose/ethanol solution, and the mixture is kneaded, extruded and dried by a standard method to give granules.

### (Formulation example 2) Tablets

| | |
|---|---|
| Sesamin-class compounds | 0.01 g |
| Carnitine | 100 g |
| Starch | 172 g |
| Sucrose fatty acid ester | 9.0 g |
| Silicon oxide | 9.0 g |

These are mixed and compressed on a single-punch tablet press into tablets having a diameter of 9 mm and a weight of 300 mg.

### (Formulation example 3) Capsules

| | |
|---|---|
| Gelatin | 60.0% |
| Glycerin | 30.0% |
| Methyl p-oxybenzoate | 0.15% |
| Propyl p-oxybenzoate | 0.51% |
| Water | q.s. |

In a soft capsule shell consisting of the ingredients above, the composition shown below was filled by a standard method to give soft capsules, each having a weight of 200 mg.

| | |
|---|---|
| Sesamin-class compounds | 10.0 mg |
| Carnitine | 100 mg |
| Glycerin fatty acid ester | 15.0 mg |
| Beeswax | 15.0 mg |
| Wheat germ oil | 160 mg |

### (Formulation example 4) Drinkable preparation

| | |
|---|---|
| Flavor: Sodium DL-tartrate | 0.1 g |
| Succinic acid | 0.009g |
| Sweetener: Liquid sugar | 800 g |
| Acidifier: Citric acid | 12 g |
| Vitamins: Vitamin C | 10 g |
| Sesamin-class compounds | 1 g |
| Carnitine | 10 g |
| Vitamin E | 30 g |
| Cyclodextrin | 5 g |
| Perfume | 15 ml |
| Potassium chloride | 1 g |
| Magnesium sulfate | 0.5 g |

The ingredients above are blended and combined with water to 10 L. About 100ml of this drinkable preparation is taken each time.

### (Formulation example 5) Tablets

A health food (tablets) containing an L-carnitine derivative designed to maintain health and/or keep in shape after slimming was prepared. L-carnitine L-tartrate (Lonza Japan Ltd.) was used as L-carnitine derivative and other functional ingredients consisting of a sesamin-class compound(s) (Takemoto Oil & Fat Co., Ltd.), Nelumbo nucifera extract powder (Matsuura Yakugyo Co., Ltd.), capsaicin (Nippon Shinyaku Co., Ltd.), caffeine (SHIRATORI Pharmaceutical Co., Ltd.), Banaba leaf extract powder (Tokiwa Phytochemical Co., Ltd.) and vitamin B1 (Tanabe Seiyaku Co., Ltd.) were added at the amounts shown below (per tablet). Sucrose fatty acid ester, cellulose, silicon dioxide, starch, and lactose were added as excipients and the mixture was compressed on a tablet press into tablets each having a diameter of 8 mm and a weight of 250 mg. It was found that no problem of hygroscopicity or deliquescence occurred during preparation and storage of the prepared tablets.

| | |
|---|---|
| L-carnitine L-tartrate | 74.3 mg |
| Sesamin-class compounds | 1.7 mg |
| Nelumbo nucifera extract powder | 16.7 mg |
| Capsaicin | 4.2 mg |
| Caffeine | 16.7 mg |
| Banaba leaf extract powder | 16.7 mg |
| Vitamin B1 | 0.18 mg |

### Example 4 Flavor test

In the following preparation examples, the sesamin-class compounds and carnitine described in Example 1 and L-carnitine L-tartrate described in Example 2 were used. Thirteen panelists were asked to lick a mixture of equal amounts of the sesamin-class compounds and carnitine (P), and L-carnitine L-tartrate (Q) in a powdery state, and to answer five questions about (1) sweetness, (2) stimulation (3) aftertaste, (4) unpalatability, (5) preference. The answers to questions (1)-(4) were chosen from the following three options: it is 1. strongly felt, 2. slightly felt, or 3. not specially felt, and the answer to (5) was chosen from either P or Q.

The results showed that more panelists preferred P to Q, as shown in Table 1 (the figures in Table 1 represent the numbers of panelists who chose each answer for P and Q). Thus, it was demonstrated that a composition of the present invention consisting of a mixture of equal amounts of a sesamin-class compound(s) and carnitine (P) is (1) slightly sweet, (2) less stimulant, (3) free of aftertaste, (4) palatable, and (5) preferred as compared with a carnitine derivative in which hygroscopicity of carnitine was improved by a conventional method, L-carnitine L-tartrate (Q), showing that the composition of the present invention is also excellent in flavor as a composition for food and beverage.

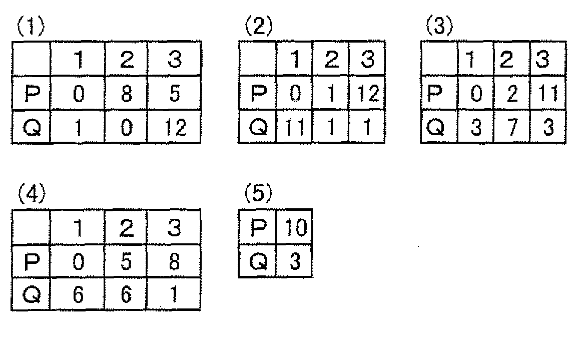

## Claims

1. A composition for food or beverage comprising a hygroscopic powdery material and a sesamin-class compound(s).

2. The composition of claim 1 wherein the hygroscopic powdery material is carnitine or a salt thereof or a derivative thereof.

3. The composition of claim 1 or 2 wherein the sesamin-class compound(s) are sesamin and/or episesamin.

4. A powdery composition for food or beverage comprising a hygroscopic powdery material and a sesamin-class compound(s) mixed with each other wherein the hygroscopic powdery material has improved hygroscopicity.

5. A food or beverage containing the composition of any one of claims 1 to 4.
